# EUROPEAN PATENT SPECIFICATION

(11) **EP 3 938 780 B1**
(45) Date of publication and mention of the grant of the patent: **05.11.2025**
(21) Application number: 19918541.4
(22) Date of filing: 05.12.2019
(51) Int. Cl.: G01N 33/49, A61B 5/15, G01N 33/72, G01N 21/29, G01N 21/78

(54) **HEMOLYSIS DETECTION BLOOD TESTING DEVICE**
BLUTTESTGERÄT FÜR HÄMOLYSENACHWEIS
DISPOSITIF DE TEST SANGUIN DE DÉTECTION D'HÉMOLYSE

(30) Priority: 12.03.2019 US 201962817144 P
(43) Date of publication of application: 19.01.2022
(73) Proprietor: Siemens Healthcare Diagnostics, Inc., Tarrytown, NY 10591 (US)
(72) Inventor: SAMPRONI, Jennifer, Braintree, Massachusetts 02184 (US); KAUFFMANN, Aaron, Elkhart, Indiana 46516 (US); LEDDEN, David, Elkhart, Indiana 46514 (US)
(74) Representative: Schweitzer, Klaus
(86) International application number: PCT/US2019/064631
(87) International publication number: WO 2020/185272

(56) References cited:
- WO-A2-2011/033000
- US-A- 5 125 415
- US-A1- 2003 236 497
- US-A1- 2013 040 333
- US-A1- 2013 040 333
- US-A1- 2014 329 268
- US-A1- 2015 153 323
- US-B2- 7 896 818
- US-B2- 9 322 761

## Description

### Background

Point-of-care testing refers generally to medical testing at or near the site of patient care, such as in an emergency room. A desired outcome of such tests is often rapid and accurate lab results to determine a next course of action in the patient care. A number of such point of care tests involve analysis of a blood sample from the patient.
Many of these tests use whole blood, plasma or serum. In these samples there may be residual broken blood cells as a result of hemolysis due to imperfections in obtaining the sample from the subject, pre-analytical blood sample handling, clinical situations (e.g., hemolytic anemia, poisons or toxins) and the whole blood separation process. In certain cases, the materials release from the hemolysed cells or cellular membrane debris can interfere with the integrity of analytical test results.

For example, if hemolysis occurs, resulting free hemoglobin in the sample may cause interference in a number of tests, thereby leading to a signal reduction, reduced measurement accuracy and precision, or to false positive results at the other end of the spectrum. For one, it has been found that the potassium concentration in a corresponding sample may increase significantly and cause a high risk of misdiagnosis in a diagnostic test for potassium levels. Hemolysis can also interfere with readings of albumin, amylase, bilirubin, calcium, magnesium, iron, phosphate, hemoglobin, haptoglobin, alkaline phosphatase, total protein, alanine aminotransferase, aspartate amino transferase, lactate dehydrogenase, creatinine kinase, cardiac troponin T, RBC, HCHC and platelet count.

### BRIEF DESCRIPTION OF THE DRAWINGS

The accompanying drawings, which are incorporated in and constitute a part of this specification, illustrate one or more implementations described herein and, together with the description, explain these implementations. In the drawings:
Figure 1 is a side elevational view of an exemplary blood testing assembly including a receptacle and a blood testing device constructed in accordance with the present disclosure.
Figure 2 is a side elevational view of the blood testing device constructed in accordance with one embodiment of the present disclosure.
Figure 3 is a top plane view of the blood testing device of Figure 2 having a color palette on the blood testing device in accordance with one embodiment of the present disclosure.
Figure 4 is a bottom plane view of the blood testing device of Figure 2 showing a connector configured to connect the blood testing device to the receptacle, such as a syringe, in accordance with the present disclosure.
Figure 5 is a top perspective, exploded view of the exemplary blood testing device of Figure 2.
Figure 6 is a bottom perspective exploded view of the exemplary blood testing device of Figure 2.
Figure 7 is a perspective, exploded view another embodiment of an exemplary blood testing assembly constructed in accordance with the present disclosure.
Figure 8 is a side elevational view of the embodiment of the blood testing assembly depicted in Figure 7.

### DETAILED DESCRIPTION

The following detailed description refers to the accompanying drawings. The same reference numbers in different drawings may identify the same or similar elements.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements, but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the inventive concept. This description should be read to include one or more and the singular also includes the plural unless it is obvious that it is meant otherwise.

Further, use of the term "plurality" is meant to convey "more than one" unless expressly stated to the contrary.

As used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

As used herein, the term "substantially" means that the subsequently described parameter, event, or circumstance completely occurs or that the subsequently described parameter, event, or circumstance occurs to a great extent or degree. For example, the term "substantially" means that the subsequently described parameter, event, or circumstance occurs at least 90% of the time, or at least 91%, or at least 92%, or at least 93%, or at least 94%, or at least 95%, or at least 96%, or at least 97%, or at least 98%, or at least 99%, of the time, or means that the dimension or measurement is within at least 90%, or at least 91%, or at least 92%, or at least 93%, or at least 94%, or at least 95%, or at least 96%, or at least 97%, or at least 98%, or at least 99%, of the referenced dimension or measurement.

To determine whether hemolysis has occurred, a number of tests have been developed. One common reagent used for determining Hb levels in a blood sample is referred to as Drabkin's Reagent. Drabkin's Reagent comprises a mixture of sodium bicarbonate, potassium ferricyanide, and potassium cyanide that lyses red blood cells and quantitatively converts all Hb in a sample into one form, cyanomethaemoglobin, which is then measured on a spectrometer using a single wavelength.

To process a sample with Drabkin's Regent, a spectrophotometer is set to 540 nm and absorbance is blanked to a water reference. Test tubes are prepared for a water reference, and samples. Drabkin's Solution (5 mL) is added to each test tube. Sample (20 µL) is added to test tubes as needed and pipetted up and down multiple times to lyse the sample. The sample is left to set for 15 minute depending on ambient conditions to convert to cyanomethaemoglobin. The absorbance at 540 nm of the sample is then read after blanking to water. The results are then interpreted with a calibration curve. Standard solutions may not need to equilibrate for 15 minutes, but will still take time requiring manual handling and result estimations.

Drabkin's Reagent does not provide a realistic picture of the extent of free Hb present at a particular point in time in a sample, which is indicative of hemolysis. Drabkin's reagent also does not indicate whether a drawn blood sample has been lysed.

Some rapid point of care hemolysis detection tests are described in patent publications. WO 2015/191450 describes techniques for detecting hemolysis using a chromatographic detection pad. US patent application no. 2017/0248618 describes techniques for detecting hemolysis by using a membrane to separate blood from plasma and then determining a color of the plasma.

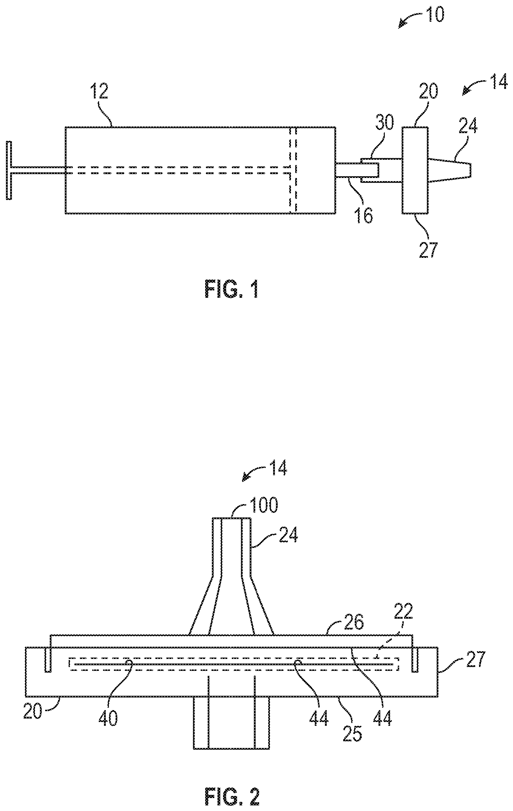

US 2013/0040333 A1 discloses a device for visual detection of hemolysis in a whole blood sample. The device is placed mainly inside a whole blood container. It comprises a filter (4) for separating blood plasma, a transfer channel (7) and a detection chamber (6) (Fig. 2a). The color of the plasma inside the detection chamber (6) can be visually inspected. Inside the detection chamber, a reagent may be deposited which reacts with hemoglobin and produces a color that indicates hemolysis. The device may be provided with a color reference for comparison with the sample plasma. The color reference may be arranged next to the visible detection chamber (6).

US 2014/0329268 A1 is directed to a device for visual detection of hemolysis in a whole blood sample from a pierceable container. The device may comprise a first and a second filter, arranged adjacent to each other. The first filter is a separation filter (40) which stops the passage of red blood cells but allows the passage of blood plasma. The second filter is a detection filter (41) which is visible through the transparent cover material of the detection compartment (6) of the device. Reagents (e.g. Dabkin's reagent) may be deposited inside the detection compartment (6). The reagents show a change of color in case hemolysis has occurred.

US 2015/0153323 A1 discloses a filtering device for separating plasma from whole blood. The device comprises a layered filter, a stopping membrane and a lateral grid. On the output side a capillary adaptor is provided for connecting to the sample input device of an analyzer.

An analyzer sold under the trademark Cholestech separates the plasma from the blood along the length of a filter membrane (lateral flow); and then presses reagent pads on a portion of the membrane to pull a sample. The analyzer sold under the trademark Cholestech does not detect hemolysis but may provide an error message if the sample is hemolyzed.

Techniques for hemolysis detection are also described in the article "Membrane-Based, Sedimentation-Assisted Plasma Separator for Point of Care Applications", Changchun Liu et al. Analytical Chemistry 2013 85(21), 10463-10470. The techniques described in this article, however, requires a large sample volume, long wait time, and secondary steps for hemolysis detection and quantification.

U.S. Patent Nos. 7,896,818; and 8,444,621 disclose a sampler cap which may be used to transfer a test sample to an analyzer without removing the sampler cap from a sampler. The sampler is a syringe, in a preferred embodiment. The sampler cap, however, does not include any manner of determining whether hemolysis has occurred in the sample. Thus, when the sampler cap is used as specified in U.S. Patent Nos. 7,896,818; and 8,444,621 to transfer a blood sample from a patient into an analyzer, hemolyzed blood may be transferred into the analyzer, which may cause interference in a number of tests.

A need exists for rapid, point-of-care testing of a blood sample to determine whether hemolysis has occurred that overcomes the shortcomings of the present testing regimes.

In accordance with one aspect, there are provided devices, systems, and processes for determining a presence of hemolysis in a sample suspected of having hemolysis (i.e., broken cell fragment, hemoglobin, etc.). Advantageously, devices, systems, and processes described herein determine whether hemolysis has occurred in a sample based upon a colorimetry assessment of a portion of the sample. The sample may be one which could potentially create a false positive result in an assay from potassium levels by providing potassium levels which are significantly larger than the associated subject's actual potassium levels in the absence of hemolysis. For example, potassium levels inside red blood cells may be 25 times higher than in plasma. Thus, if hemolysis occurs, the potassium value of the sample in question may be increased significantly. When a subject's potassium levels are not actually as high as indicated, a false positive result may in turn result in misdiagnosis and mistreatment of a disorder characterized by elevated potassium levels. For example, as a result of hemolysis, a subject might be misdiagnosed with having hyperkalemia or any other disorder or condition characterized by elevated potassium levels, e.g., Addison's disease or hemolytic anemia. Further, the subject may be misdiagnosed as having elevated potassium levels as a side effect of taking medications such as water pills (diuretics) or blood pressure drugs and unnecessarily instructed to cease taking such medications to the subject's detriment. In addition, a false positive result could inadvertently lead to one unnecessarily being provided with agents to remove potassium from the intestines before potassium is absorbed or other unnecessary treatments. Thus, advantageously, the blood testing device described herein can be utilized in a screening process for an unacceptable level of hemolysis prior to analysis of the sample for potassium levels or for confirming the integrity of test results already run.

In certain embodiments, the sample is a whole blood sample which includes a quantity of whole blood cells, including red blood cells, white blood cells, and platelets. Within the sample, the extent of hemolysis may correlate to an amount of hemoglobin therein. As used herein, it is understood that the term "hemoglobin" refers to any and all hemoglobin molecules obtained from drawn blood. Hemoglobin is commonly known as the oxygen-carrying pigment and predominant protein of red blood cells. Hemoglobin is composed of four protein chains, two alpha chains and two beta chains, each with a ring-like heme group containing an iron atom. Oxygen binds reversibly to these iron atoms. In its oxygenated state, hemoglobin may be referred to as oxyhemoglobin and is characterized by a bright red. In the reduced state, hemoglobin may be referred to as deoxyhemoglobin and is characterized by a purple-blue color.

In accordance with another aspect, there are provided devices, systems, and processes for a blood collection assembly having a hemolysis indicating feature.

In accordance with another aspect, there are provided blood testing devices, systems, accessories and processes having a plasma separating feature.

In accordance with another aspect, there are provided blood testing devices, systems, accessories, and processes having a hemolysis indicating feature, as claimed in claim 1, drawn to a blood testing device, claim 10, drawn to a blood testing assembly, and claim 11, drawn to a method for determining degree of hemolysis.

Referring now to the Figures and in particular to Figure 1, shown therein is a diagrammatic view of a blood testing assembly 10 constructed in accordance with the present disclosure. In general, the blood testing assembly 10 includes a receptacle 12 containing a sample of blood, and a blood testing device 14. The receptacle 12 has a port 16 configured to transfer the blood out of the receptacle 12. The blood testing device 14 has a housing 20 constructed of a fluid impermeable material. The housing 20 has an interior space 22 which is shown by dashed lines in Fig. 2. The housing 20 may also include a vent 24 configured to allow gas to escape from the interior space 22, and prevent liquid (i.e., plasma) from escaping from the interior space 22. In another embodiment, the housing 20 may be devoid of the vent 24. This can be accomplished by pressurizing the interior space 22 within the housing 20 below atmospheric pressure (e.g., a vacuum) so that the housing 20 draws the sample of blood into the interior space 22. The housing 20 has a top wall 25, a bottom wall 26, and a sidewall 27 extending between the top wall 25 and the bottom wall 26. The vent 24 extends from the bottom wall 26. The blood testing device 14 also includes a receptacle connector 30 extending from the top wall 25 that is connected to the housing 20, and the port 16 of the receptacle 12. The receptacle connector 30, in one embodiment, is a tubular member that forms a passage from the port 16 (outside of the housing 20) to the interior space 22 so that at least a portion (e.g., 0.5 - 1 mL) of the sample of blood can be transferred from the receptacle to the interior space 22. Smaller volumes of blood are possible dependent upon the design of the housing 20.

In one embodiment, the receptacle 12 is a syringe, and the receptacle connector 30 is a device known in the art as a luer lock. It should be understood that the receptacle 12 and the receptacle connector 30 can be in other forms. For example, the receptacle 12 may be a device known in the art as a vacutainer that can be used for collecting blood and transporting the blood for purposes of testing. In another embodiment, the receptacle 12 can be simultaneously connected to an instrument, such as a blood gas analyzer, and the blood testing device 14. For example, the housing 20 and the vent 24 can be constructed in a manner described for constructing the hollow body, and the analyzer connector 162 (in U.S. Patent No. 7,896,818) to provide a venting mechanism (e.g., venting conduit) and an analyzer connector for attachment to an analyzer. This permits the analyzer connector of the blood testing device 14 to be connected to the analyzer, and used for transferring the sample from the receptacle 12 to the analyzer through the blood testing device 14. In one embodiment, the analyzer may have an analyzer probe that extends through the analyzer connector and into the receptacle 12 to obtain a sample directly from the receptacle 12. The blood may be collected from an animal, such as a human, or a non-human (such as a cat, dog, cow, horse, fish, or the like).

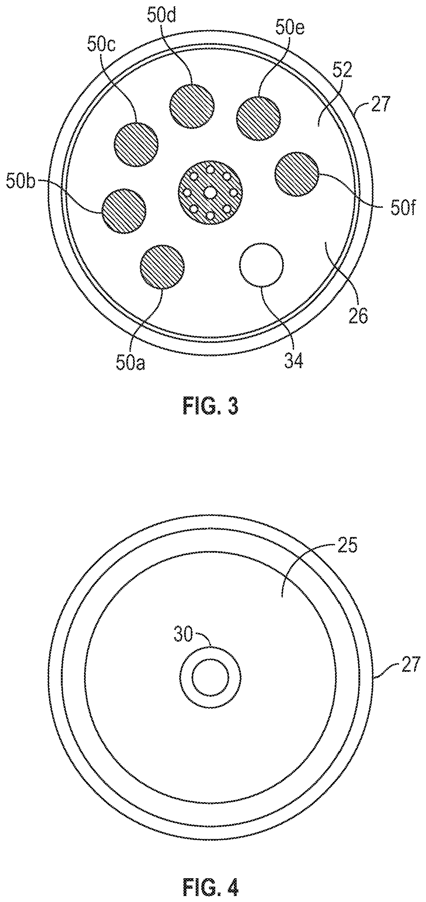

Shown in Figure 2 is a side elevational view of an exemplary blood testing device 14 constructed in accordance with the present disclosure. The housing 20 is constructed of fluid impermeable material so that the housing 20 can hold and contain a sample of blood containing blood cells, suspended within plasma. The housing 20 is shown as having a cylindrical shape, but it should be understood that the housing 20 can be provided with any shape, such as a square shape, round shape, rectangular shape, offset rectangular shape, triangular shape, offset triangular shape, or the like. The housing 20 includes a window 34 (See Figure 3) constructed of an optically transparent material that is configured to pass light bidirectionally from outside of the housing 20 to the interior space 22, and from the interior space 22 to the outside of the housing 20. In one embodiment, the light is in a visible part of the electromagnetic spectrum to allow a user to look through the window 34 and into the interior space 22 of the housing 20. As shown in Figure 3, the window 34 can be a transparent portion of the bottom wall 26, although in other embodiments the window can be located on the sidewall 27 of the housing 20.

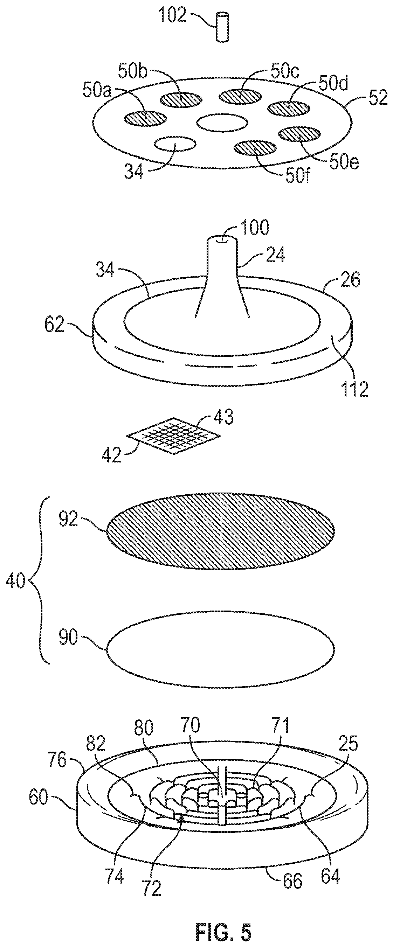

The blood testing device 14 is also provided with a separator 40 (Figure 5) within the housing 20, and a reagent 42 (Figure 5). The separator 40 is configured to receive blood having blood cells and plasma, separate the blood cells from the plasma, and direct the plasma to an inspection zone 44 within the interior space 22. The reagent 42 is positioned within the inspection zone 44 and adjacent to the window 34 so that the reagent 42 is visible through the window 34. The reagent 42 is configured to change colors in the presence of hemoglobin within the plasma to provide an indication of a state of hemolysis of the blood. When the blood testing device 14 is configured with an analyzer connector configured to be attached to the analyzer discussed above, the analyzer inlet probe may approach the separator 40 and pierce the separator 40 to obtain access to the sample within the receptacle 12.

In one embodiment, the reagent 42 includes a pad 43 (see Figure 5) that changes color due to an amount of hemoglobin within the plasma. The pad 43 may be a hydrophilic membrane for effecting a pinking color change so that a user or an external reader device can colorimetrically analyze and correlate the color change to a color (e.g., visual) reference. The pad 43 may be white or other color. In some embodiments, the pad 43 can be made of cellulose, nitrocellulose, carboxymethylcellulose, or other material that will tend to retain protein.

The color change of the pad 43 is visible through the window 34 to permit a user or external reader device to view the reagent 42 and compare the color of the reagent 42 to regions 50a-f of a color palette 52. Each of the regions 50a-f has a different color that has been correlated with the reagent 42 to indicate a different state of hemolysis, e.g., an amount of hemoglobin within the plasma. The amount of hemoglobin can be determined by the user comparing the color of the reagent 42 to the regions 50a-f in the color palette 52. As will be discussed below, the color palette 52 can be a sticker having a bonding material connecting a transparent substrate to the bottom wall 26, and surrounding the vent 24. In one embodiment, the regions 50a-f are provided in a circular pattern that is coaxial with the vent 24. It should be understood, however, that other configurations of the regions 50a-f can be used. Further, the sticker can have an opening aligned with the window 34 to permit the user to view the reagent 42. In another example, the window 34 may be a transparent region of the sticker. In this embodiment, the housing 20 (or a portion thereof) may be opaque, and have an opening aligned with the pad 43. The sticker may be connected to the housing 20 to form a fluid impermeable seal. In this embodiment, a user or external reader may view the pad 43 through the transparent region of the sticker and the opening.

Exemplary reagents 42 that can be used, and resulting color changes, are set forth below in Table 1.

**Table 1**

| Assay Purpose (and simplified formula) | Approximate TTR |
|---|---|
| Membrane (color change) | 30 seconds |
| Blood: This test is based on the peroxidase-like activity of hemoglobin which catalyzes the reaction of diisopropylbenzene di hydroperoxide (w/w 6.8%) and 3,3',5,5'tetramethylbenzidine (w/w 4%). The resulting color ranges from orange through green. Very high levels may continue color development to blue | <60 seconds |
| Protein-Low (Albumin): This test is based on dye binding using a high affinity sulfonephthalein dye. At a constant pH, the development of any color ranging from pale green to aqua blue. | <50 seconds |
| Ingredients: 1.9% w/w bis (3',3"-diiodo-4',4"-dihydroxy-5',5"-dinitrophenyl)-3,4,5,6-tetrabromosulfonephthalein | |
| Protein-High: This test is based on the protein-error-of-indicators principle. At a constant pH, the development of any green color is due to the presence of protein. Controls range from yellow, low-green, green, and green-blue. | <50 seconds |
| Ingredients: 0.3% w/w tetrabromphenol blue | |

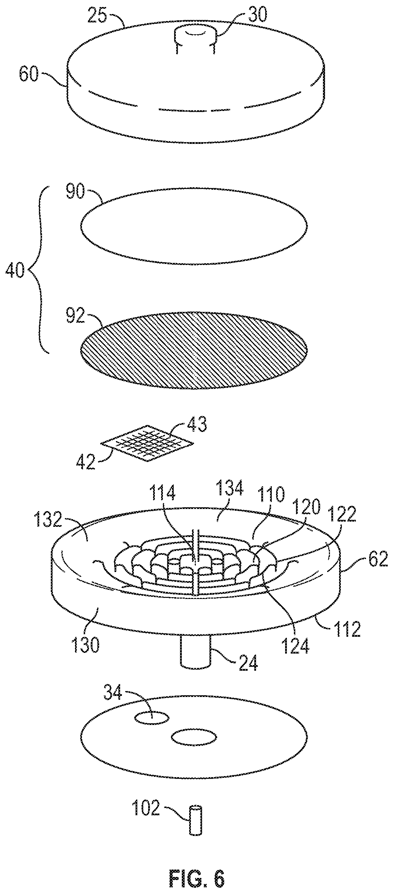

Referring now to Figures 5 and 6, shown therein are exploded views of the exemplary embodiment of the blood testing device 14. In this example, the housing 20 is provided with a housing top 60 and a housing bottom 62 that are connected together to enclose the interior space 22. The housing top 60 includes the top wall 25. The housing top 60 can be integrally formed as a unitary structure, or can be formed by separate components which are joined together via any suitable methodology such as sonic welding or a bonding material, such as an adhesive or a cohesive.

The top wall 25 is provided with an interior surface 64, and an exterior surface 66 generally opposite the interior surface 64. The top wall 25 is also provided with an opening 70 that communicates with the receptacle connector 30 to permit the blood to enter the interior space 22 of the housing 20. In one embodiment, the opening 70 can be located in a central region of the top wall 25 as shown in Figure 5. It should be understood that the opening 70 can be placed in other parts of the top wall 25 so long as the opening 70 communicates with the receptacle connector 30. The interior surface 64 is shaped so as to diffuse the blood from the opening 70 over a separation area 71 that, in this example, encompasses a substantial part of the interior surface 64. For example, the interior surface 64 can be shaped to include a plurality of channels 72 and ribs 74. The housing top 60 also includes a sidewall portion 76 that extends from the top wall 25 and forms a portion of the sidewall 27 when the housing top 60 and the housing bottom 62 are connected together. The interior surface 64 and the sidewall portion 76 have a cup shape forming a recess 80. The interior surface 64 may also be provided with a perimeter region 82 surrounding the separation area 71. As will be discussed below, the perimeter region 82 is shaped so as to mate with the separator 40. For example, the perimeter region 82 and the separator 40 may both be planar.

The housing top 60 may be formed from any suitable liquid impermeable material that is also inert to at least hemoglobin. For example, without limitation, the housing top 60 may be formed from a material comprising polystyrene, polyethylene, polycarbonate, polypropylene, fluoropolymer, polyester, glass, metals, ceramics, suitable composite materials, and combinations thereof as would be appreciated by those skilled in art. Further, the housing top 60 may be constructed of a material that is opaque to light in the visible part of the electromagnetic spectrum.

The separator 40 may be provided with one or more stacked filters. In the example shown, the separator 40 is provided with a first filter 90 and a second filter 92 that are aligned, and stacked one on top of the other. The first filter 90 and the second filter 92 may be the same size and shape, e.g., in this case both the first filter 90 and the second filter 92 are circular. In other embodiments, the first filter 90 and the second filter 92 may be different sizes and/or shapes. In the disclosed embodiment, the first filter 90 is positioned on and engages the interior surface 64, and the second filter 92 is positioned on and engages the first filter 90.

The first filter 90 may be designed to separate the blood cells in the blood from the plasma, and then to pass the plasma to the second filter 92. For example, in the embodiment shown in Figure 5, the first filter 90 may isolate plasma and hemolysis products, e.g., hemoglobin, from whole blood cells in a sample such as a whole blood sample. In an embodiment, the first filter 90 comprises a plasma separation membrane as is commercially available in the art. In certain embodiments, the plasma separation membrane comprises an asymmetric material, which is able to retain a plurality of whole blood cells thereon while allowing plasma and small molecules/complexes to travel there through. A number of different plasma separation membranes are commercially available and may be suitable for use in the blood testing device 14. For example, the plasma separation membrane may comprise an asymmetric polysulfone material as is commercially available from Pall Corporation (currently under the trademark Vivid^{™}). Alternatively, the first filter 90 may comprise any other suitable material or device that can provide a sample comprising plasma and components from hemolysis (if present) therein.

The plasma separated by the first filter 90 is provided to the second filter 92. The second filter 92 is provided with a predetermined color and forms a background or setting for the reagent 42. The second filter 92 overlaps the reagent 42. In one embodiment, the reagent 42 is positioned between the second filter 92 and the window 34 so that the second filter 92 provides a background color for the reagent 42. When blood that is suspected as having hemolysis is provided into the interior space 22 through the receptacle connector 30 and the opening 70, the blood is diffused by the separation area 71 and passes through the first filter 90. The first filter 90 separates the blood cells and platelets from the plasma, and then passes the plasma to the second filter 92. The plasma saturates the second filter 92 and passes through the second filter 92 to the reagent 42. The reagent 42 reacts with the plasma and may change color to indicate a state of hemolysis, or an unacceptable level of hemolysis. The second filter 92 provides a consistent color background, and therefore assists with the colorimetric comparison of the color of the reagent 42. In one example, the second filter 92 is black filter paper, although it should be understood that other colors could be used. In one embodiment, the first filter 90 can be a predetermined color to provide a consistent color background. In this embodiment, the second filter 92 may be transparent, or eliminated.

As discussed above, the vent 24 is designed to permit gas to pass through the vent 24, but prevent the passage of a fluid through the vent 24. In one embodiment, the vent 24 is a tubular member having an interior opening 100. The vent 24 includes an overflow plug 102 positioned within and blocking the interior opening 100. The overflow plug 102 is constructed of a material capable of venting gas from the interior space 22, but preventing the passage of the fluid through the vent 24. Exemplary materials for making the overflow plug 102 include starch, cellulose, and teflon.

The housing bottom 62 includes the bottom wall 26. The bottom wall 26 is provided with an interior surface 110, and an exterior surface 112 generally opposite the interior surface 110. The bottom wall 26 is also provided with an opening 114 that communicates with the interior opening 100 of the vent 24 to permit gas and fluid to enter the interior opening 100 from the interior space 22 of the housing 20. In one embodiment, the opening 114 can be located in a central region of the bottom wall 26 as shown in Figure 6. It should be understood that the opening 114 can be placed in other parts of the bottom wall 26 so long as the opening 114 communicates with the interior opening 100 of the vent 24. The interior surface 110 is shaped so as to diffuse the plasma from the second filter 92 over a reagent saturation area 120 that, in this example, encompasses a substantial part of the interior surface 110. For example, the interior surface 110 can be shaped to include a plurality of channels 122 and ribs 124. The housing bottom 62 also includes a sidewall portion 130 that extends from the bottom wall 26 and forms a portion of the sidewall 27 when the housing top 60 and the housing bottom 62 are connected together. The interior surface 110 and the sidewall portion 130 may have a cup shape forming a recess 132. The interior surface 110 may also be provided with a perimeter region 134 surrounding the reagent saturation area 120. As will be discussed below, the perimeter region 134 is shaped so as to mate with the separator 40. For example, the perimeter region 134 may be planar.

The housing bottom 62 may be formed from any suitable liquid impermeable material that is also inert to at least hemoglobin. For example, without limitation, the housing top 60 may be formed from a material comprising polystyrene, polyethylene, polycarbonate, polypropylene, fluoropolymer, polyester, glass, metals, ceramics, suitable composite materials, and combinations thereof as would be appreciated by those skilled in art. Further, a portion of the housing bottom 62 forming the window 34 is constructed of a material that is transparent to light in the visible part of the electromagnetic spectrum.

In operation, a sample of blood to be tested is placed within the receptacle 12. The receptacle connector 30 of the blood testing device 14 may be connected to the port 16 of the receptacle 12, and an amount of blood transferred through the port 16 and the receptacle connector 30 into the interior space 22 of the blood testing device 14. As the blood is transferred into the interior space 22, air within the interior space 22 is directed through the vent 24. As the blood enters the interior space 22, the blood is diffused by the channels 72 and the ribs 74 and is applied to the first filter 90. The first filter 90 separates the blood cells and platelets from the plasma, and passes the plasma to the second filter 92. The plasma saturates the second filter 92, and passes the plasma into the reagent saturation area 120 so that the plasma can contact and saturate the reagent pad 43 of the reagent 42. The saturated reagent pad 43 then may or may not change color depending upon the state of hemolysis of the blood. In any event, the color of the reagent pad 43 can be viewed by the user, and a determination of whether the blood has hemolysis can be made by comparing the color of the reagent pad 43 to the regions 50a-f of the color palette 52. Thereafter, the blood testing device 14 may be removed from the receptacle 12 and discarded. When the blood sample does not have an unacceptable level of hemolysis, the blood sample can be tested using conventional techniques, such as providing the blood sample into a cartridge of a blood gas analyzer.

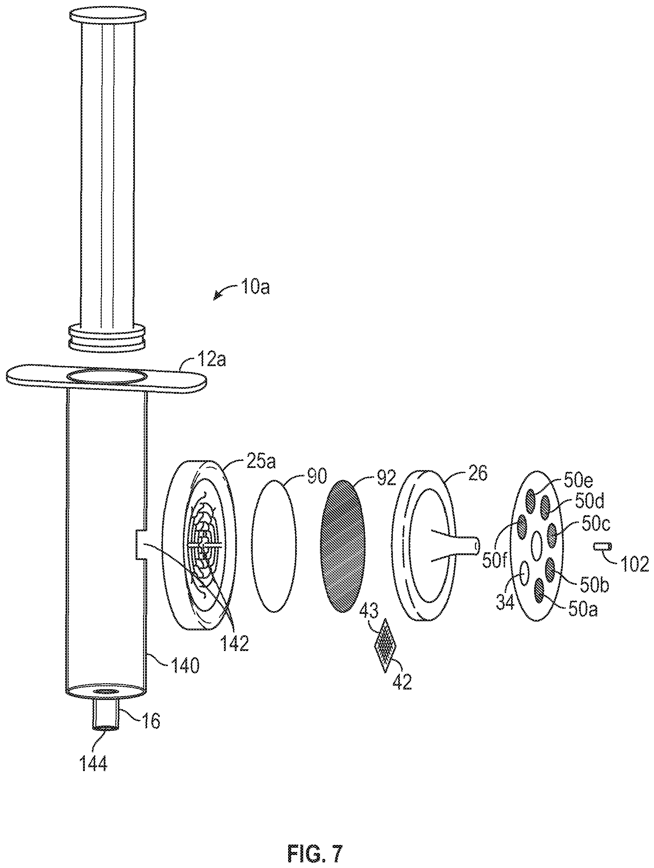

Figure 7 is a perspective, exploded view another embodiment of an exemplary blood testing assembly 10a constructed in accordance with the present disclosure. The blood testing assembly 10a includes a receptacle 12a and a blood testing assembly 14a. Similar elements between the receptacle 12 and 12a will be labeled with the same reference numerals. Likewise, similar elements between the blood testing assembly 14 and 14a will be labeled with the same reference numerals.

The receptacle 12a and the blood testing assembly 14a are similar in construction and function to the receptacle 12 and the blood testing assembly 14 described above, with the exception that the receptacle 12a and the blood testing assembly 14a are integrated into a single device which in this example is a syringe. In this regarding, the receptacle 12a has a sidewall 140, a first port 142, and a second port 144 (which in some embodiments can be the same as the port 16 described above). In the embodiment shown, the sidewall 140 and a top wall 25a of the blood testing device 14a are integrally formed as a unitary structure. The sidewall 140 and the top wall 25a include the first port 142 formed through the sidewall 140 and the top wall 25a so as to permit a sample, e.g., blood, to flow from the receptacle 12a and into the interior space 22 of the blood testing device 14a. Other than being integrally formed with the sidewall 140, the top wall 25a is identical to the top wall 25 in construction and function.

The flow of sample from the receptacle 12a and into the interior space 22 of the blood testing device 14a can be implemented in various manners. For example, the port 142 can be designed to establish capillary action between the receptacle 12a and the interior space 22. In this embodiment, after a sample is drawn through the port 144 with the port 142 (or the vent 102) closed via a valve. The valve may be opened, and the sample of blood may displace the gas in the interior space 22 of the housing 20 through capillary action. In this case, the volume of sample is limited by a volume of the interior space 22 that is not filled with the first membrane 90, the second membrane 92, and the reagent pad 43. In either implementation, any overfill should be stopped by the overflow plug 102.

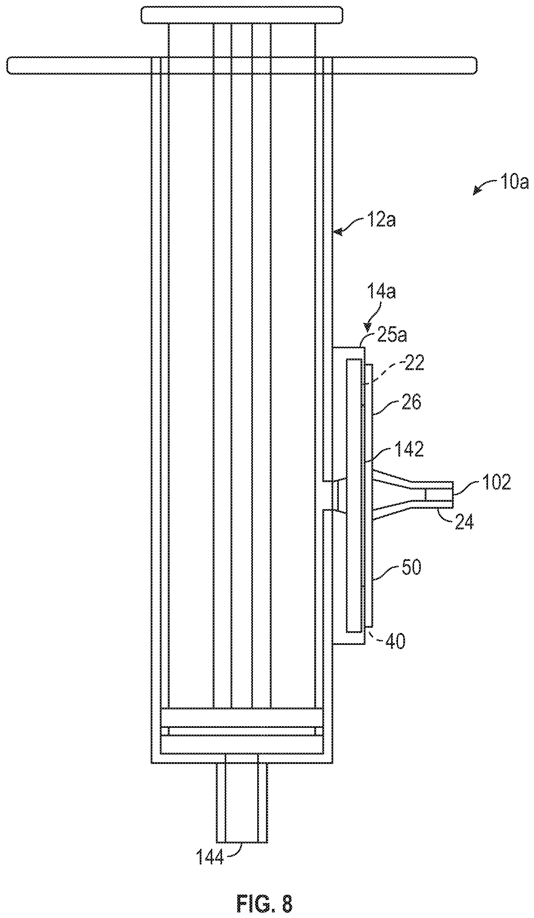

Figure 8 is a side elevational view of the embodiment of the blood testing assembly 10a depicted in Figure 7.

The operation of the blood testing assembly 10a is identical to the operation of the blood testing assembly 10 described above, with the exception that the blood testing assembly 14a does not have a receptacle connector 30 that needs to be connected to the port 16 of the receptacle 12 due to the receptacle 12a and the blood testing assembly 14a being integral and a single device. Further, in the embodiments that include a valve being positioned within the first port 142, the operation may include opening the valve to permit the sample to flow through the first port 142 and into the interior space 22, and closing the valve to prevent any further sample from flowing through the first port 142.

From the above description, it is clear that the inventive concepts disclosed herein are well adapted to carry out the objects and to attain the advantages mentioned herein as well as those inherent in the inventive concepts disclosed herein. While presently preferred embodiments of the inventive concepts disclosed herein have been described for purposes of this disclosure, it will be understood that numerous changes may be made, limited only by the scope of the appended claims.

## Claims

1. A blood testing device (14), comprising:
a housing (20) constructed of a fluid impermeable material, the housing having an interior space (22), and a window (34) constructed of an optically transparent material that is configured to pass light bidirectionally from outside of the housing (20) to the interior space (22), and from the interior space to the outside of the housing (20), the light being in a visible part of an electromagnetic spectrum,
a separator (40) within the housing, the separator configured to receive blood having blood cells and plasma, separate the blood cells from the plasma, and direct the plasma to an inspection zone within the interior space;
a reagent (42) within the inspection zone (44) and adjacent to the window (34) so that the reagent is visible through the window (34), the reagent (42) configured to change colors in the presence of hemoglobin within the plasma to provide an indication of a state of hemolysis of the blood,
**characterized in that** the housing (20) comprises a vent configured to allow gas to escape from the interior space (22) and prevent liquid from escaping from the interior space; wherein the vent (24) has a tubular interior opening (100) and includes an overflow plug (102), positioned within and blocking the interior opening (100), the overflow plug (102) being constructed of a material capable of venting gas from the interior space (22), but preventing the passage of the fluid through the vent (24), that material preferably being starch, cellulose or teflon.

2. The blood testing device of claim 1, wherein the separator includes a plasma separating membrane (90).

3. The blood testing device of claim 2, wherein the plasma separating membrane (90) is provided with a predetermined color so as to form a backdrop for the reagent (42).

4. The blood testing device of claim 1, wherein the separator includes a first filter and a second filter that are adjacently disposed and overlapping, one of the first filter and the second filter being a plasma separation membrane, and the other one of the first filter and the second filter (92) is provided with a predetermined color so as to form a backdrop for the reagent (42).

5. The blood testing device of claim 1, wherein the separator (40) includes a first filter (90) stacked with a second filter (92), the first filter being a plasma separating membrane (90), the second filter being between the first filter and the reagent (42), and overlapping the reagent so as to form a backdrop for the reagent when the reagent is viewed through the window.

6. The blood testing device of claim 1, further comprising a color palette (52) having a plurality of regions (50a - 50f) being a color of the reagent correlated to indicate a predetermined state of hemolysis of a blood sample, the color palette being on the housing (20).

7. The blood testing device of claim 6, wherein the color palette (52) is a sticker having a bonding material connecting a substrate to the housing (20), the color regions being supported by the substrate.

8. The blood testing device of claim 6, wherein the color palette (52) surrounds the vent (24).

9. The blood testing device of claim 1, further comprising a receptacle connector connected to the housing (20) and a port of a receptacle, the receptacle connector forming a passage from the port, outside of the housing, to the interior space (22).

10. The blood testing device of claim 1, wherein the housing (20) includes an analyzer connector configured to connect the housing to an analyzer.

11. A blood testing assembly, comprising:
a receptacle containing blood, and having a port configured to transfer the blood out of the receptacle; and
the blood testing device according to any of claims 1-10.

12. A method, comprising:
connecting a blood testing device (14) having a plasma separation membrane (90) and a reagent, as claimed in any one of claims 1 to 10, to a syringe containing blood having blood cells and plasma;
passing a blood sample of the blood from the syringe through a plasma separation membrane within the blood testing device to separate the plasma from the blood cells;
saturating the reagent with the plasma; and
colorimetrically analyzing the reagent to determine a degree of hemolysis within the blood sample.

13. The method of claim 12, further comprising obtaining a sample of the blood sample by an analyzer through the blood testing device (14), comprising the step of removal of plug (102).

14. The method of claim 13, wherein obtaining the sample includes penetrating the plasma separation membrane (90) with an analyzer inlet probe of an analyzer.

## Patentansprüche

1. Bluttestvorrichtung (14), umfassend:
ein Gehäuse (20), das aus einem flüssigkeitsundurchlässigen Material gefertigt ist, wobei das Gehäuse einen Innenraum (22) und ein Fenster (34) aufweist, das aus einem optisch transparenten Material gefertigt ist, das so ausgelegt ist, dass es Licht bidirektional von der Außenseite des Gehäuses (20) zu dem Innenraum (22) und vom Innenraum zur Außenseite des Gehäuses (20) leitet, wobei sich das Licht in einem sichtbaren Teil eines elektromagnetischen Spektrums befindet,
einen Separator (40) innerhalb des Gehäuses, wobei der Separator dazu ausgelegt ist, Blut mit Blutzellen und Plasma aufzunehmen, die Blutzellen vom Plasma zu trennen und das Plasma auf eine Inspektionszone innerhalb des Innenraums zu lenken;
ein Reagenz (42) innerhalb der Inspektionszone (44) und neben dem Fenster (34), so dass das Reagenz durch das Fenster (34) sichtbar ist, wobei das Reagenz (42) dazu ausgelegt ist, in Gegenwart von Hämoglobin im Plasma seine Farbe zu ändern, um einen Hinweis auf einen Zustand der Hämolyse des Blutes zu liefern,
**dadurch gekennzeichnet, dass** das Gehäuse (20) eine Entlüftung aufweist, die dazu ausgelegt ist, es Gas zu ermöglichen, aus dem Innenraum (22) zu entweichen und das Entweichen von Flüssigkeit aus dem Innenraum zu verhindern; wobei die Entlüftung (24) eine röhrenförmige Innenöffnung (100) aufweist und einen Überlaufstopfen (102) aufweist, der innerhalb der Innenöffnung (100) positioniert ist und diese blockiert, wobei der Überlaufstopfen (102) aus einem Material gefertigt ist, das in der Lage ist, Gas aus dem Innenraum (22) abzulassen, aber den Durchgang der Flüssigkeit durch die Entlüftung (24) zu verhindern, wobei dieses Material vorzugsweise Stärke, Zellulose oder Teflon ist.

2. Bluttestvorrichtung nach Anspruch 1, wobei der Separator eine Plasmatrennmembran (90) aufweist.

3. Bluttestvorrichtung nach Anspruch 2, wobei die Plasmatrennmembran (90) eine vorgegebene Farbe aufweist, um einen Hintergrund für das Reagenz (42) zu bilden.

4. Bluttestvorrichtung nach Anspruch 1, wobei der Separator einen ersten Filter und einen zweiten Filter aufweist, die nebeneinander angeordnet sind und sich überlappen, wobei einer des ersten Filters und des zweiten Filters eine Plasmatrennmembran ist, und der andere des ersten Filters und des zweiten Filters (92) eine vorgegebene Farbe aufweist, um einen Hintergrund für das Reagenz (42) zu bilden.

5. Bluttestvorrichtung nach Anspruch 1, wobei der Separator (40) einen ersten Filter (90) aufweist, der mit einem zweiten Filter (92) gestapelt ist, wobei der erste Filter eine Plasmatrennmembran (90) ist, der zweite Filter sich zwischen dem ersten Filter und dem Reagenz (42) befindet und das Reagenz überlappt, so dass er einen Hintergrund für das Reagenz bildet, wenn das Reagenz durch das Fenster betrachtet wird.

6. Bluttestvorrichtung nach Anspruch 1, ferner umfassend eine Farbpalette (52) mit einer Vielzahl von Regionen (50a - 50f) mit einer mit dem Reagenz korrelierenden Farbe, um einen vorgegebenen Zustand der Hämolyse einer Blutprobe anzuzeigen, wobei sich die Farbpalette auf dem Gehäuse (20) befindet.

7. Bluttestvorrichtung nach Anspruch 6, wobei die Farbpalette (52) ein Aufkleber mit einem Bindungsmaterial ist, das ein Substrat mit dem Gehäuse (20) verbindet, wobei die Farbbereiche durch das Substrat gestützt werden.

8. Bluttestvorrichtung nach Anspruch 6, wobei die Farbpalette (52) die Entlüftung (24) umgibt.

9. Bluttestvorrichtung nach Anspruch 1, ferner umfassend einen Behälterverbinder, der mit dem Gehäuse (20) verbunden ist, und eine Anschlussöffnung eines Behälters, wobei der Behälterverbinder einen Durchgang von der Anschlussöffnung außerhalb des Gehäuses zum Innenraum (22) bildet.

10. Bluttestvorrichtung nach Anspruch 1, wobei das Gehäuse (20) einen Analysatoranschluss aufweist, der dazu ausgelegt ist, das Gehäuse mit einem Analysator zu verbinden.

11. Bluttestbaugruppe, umfassend:
einen Behälter, der Blut enthält und eine Anschlussöffnung aufweist, die zum Überführen des Blutes aus dem Behälter ausgelegt ist; und
die Bluttestvorrichtung nach einem der Ansprüche 1-10.

12. Verfahren, umfassend:
Verbinden einer Bluttestvorrichtung (14), die eine Plasmatrennmembran (90) und ein Reagenz aufweist, nach einem der Ansprüche 1 bis 10, mit einer Spritze, die Blut mit Blutzellen und Plasma enthält;
Führen einer Blutprobe des Blutes aus der Spritze durch eine Plasmatrennmembran in der Bluttestvorrichtung, um das Plasma von den Blutzellen zu trennen;
Sättigen des Reagenzes mit dem Plasma; und
kolorimetrische Analyse des Reagenzes, um einen Grad der Hämolyse in der Blutprobe zu bestimmen.

13. Verfahren nach Anspruch 12, ferner umfassend das Gewinnen einer Probe der Blutprobe durch einen Analysator durch die Bluttestvorrichtung (14), umfassend den Schritt des Entfernens des Stopfens (102).

14. Verfahren nach Anspruch 13, wobei das Gewinnen der Probe das Durchdringen der Plasmatrennmembran (90) mit einer Analysatoreinlasssonde eines Analysators umfasst.

## Revendications

1. Dispositif de test sanguin (14), comprenant :
un boîtier (20) construit d'un matériau imperméable aux fluides, le boîtier ayant un espace intérieur (22), et une fenêtre (34) construite d'un matériau optiquement transparent qui est configuré pour faire passer la lumière de manière bidirectionnelle de l'extérieur du boîtier (20) à l'espace intérieur (22), et de l'espace intérieur à l'extérieur du boîtier (20), la lumière se trouvant dans une partie visible du spectre électromagnétique,
un séparateur (40) à l'intérieur du boîtier, le séparateur étant configuré pour recevoir du sang ayant des cellules sanguines et du plasma, séparer les cellules sanguines du plasma et diriger le plasma vers une zone d'inspection à l'intérieur de l'espace intérieur ;
un réactif (42) dans la zone d'inspection (44) et adjacent à la fenêtre (34) de manière à ce que le réactif soit visible à travers la fenêtre (34), le réactif (42) étant configuré pour changer de couleur en présence d'hémoglobine dans le plasma afin de fournir une indication de l'état d'hémolyse du sang,
**caractérisé en ce que** le boîtier (20) comprend un évent configuré pour permettre au gaz de s'échapper de l'espace intérieur (22) et empêcher le liquide de s'échapper de l'espace intérieur ; dans lequel l'évent (24) a une ouverture intérieure tubulaire (100) et
comprend un bouchon de trop-plein (102), positionné à l'intérieur et bloquant l'ouverture intérieure (100), le bouchon de trop-plein (102) étant construit dans un matériau capable d'évacuer le gaz de l'espace intérieur (22), mais empêchant le passage du fluide à travers l'évent (24), ce matériau étant de préférence de l'amidon, de la cellulose ou du Téflon.

2. Dispositif de test sanguin de la revendication 1, dans lequel le séparateur comprend une membrane de séparation de plasma (90).

3. Dispositif de test sanguin de la revendication 2, dans lequel la membrane de séparation de plasma (90) est pourvue d'une couleur prédéterminée de manière à former un fond pour le réactif (42).

4. Dispositif de test sanguin de la revendication 1, dans lequel le séparateur comprend un premier filtre et un deuxième filtre disposés de manière adjacente et se chevauchant, l'un du premier filtre et du deuxième filtre étant une membrane de séparation de plasma, et l'autre du premier filtre et du deuxième filtre (92) étant pourvu d'une couleur prédéterminée de manière à former un fond pour le réactif (42).

5. Dispositif de test sanguin de la revendication 1, dans lequel le séparateur (40) comprend un premier filtre (90) empilé avec un deuxième filtre (92), le premier filtre étant une membrane de séparation de plasma (90), le deuxième filtre étant entre le premier filtre et le réactif (42), et chevauchant le réactif de manière à former un fond pour le réactif lorsque le réactif est vu à travers la fenêtre.

6. Dispositif de test sanguin de la revendication 1, comprenant en outre une palette de couleurs (52) ayant une pluralité de régions (50a - 50f) représentant une couleur du réactif corrélée pour indiquer un état prédéterminé d'hémolyse d'un échantillon de sang, la palette de couleurs étant sur le boîtier (20).

7. Dispositif de test sanguin de la revendication 6, dans lequel la palette de couleurs (52) est un autocollant ayant un matériau de liaison reliant un substrat au boîtier (20), les régions de couleur étant supportées par le substrat.

8. Dispositif de test sanguin de la revendication 6, dans lequel la palette de couleurs (52) entoure l'évent (24).

9. Dispositif de test sanguin de la revendication 1, comprenant en outre un connecteur de réceptacle connecté au boîtier (20) et à un port d'un réceptacle, le connecteur de réceptacle formant un passage du port, à l'extérieur du boîtier, à l'espace intérieur (22).

10. Dispositif de test sanguin de la revendication 1, dans lequel le boîtier (20) comprend un connecteur d'analyseur configuré pour connecter le boîtier à un analyseur.

11. Ensemble de test sanguin comprenant
un réceptacle contenant du sang et ayant un port configuré pour transférer le sang hors du réceptacle ; et
le dispositif de test sanguin selon l'une quelconque des revendications 1 à 10.

12. Procédé comprenant les étapes consistant à :
connecter un dispositif de test sanguin (14) ayant une membrane de séparation de plasma (90) et un réactif, selon l'une quelconque des revendications 1 à 10, à une seringue contenant du sang avec des cellules sanguines et du plasma ;
faire passer un échantillon de sang provenant de la seringue à travers une membrane de séparation de plasma à l'intérieur du dispositif de test sanguin pour séparer le plasma des cellules sanguines ;
saturer le réactif avec le plasma ; et
analyser colorimétriquement le réactif pour déterminer le degré d'hémolyse de l'échantillon de sang.

13. Procédé de la revendication 12, comprenant en outre l'obtention d'un échantillon de l'échantillon de sang par un analyseur à travers le dispositif de test sanguin (14), comprenant l'étape de retrait du bouchon (102).

14. Procédé de la revendication 13, dans lequel l'obtention de l'échantillon comprend la pénétration de la membrane de séparation de plasma (90) avec une sonde d'entrée d'analyseur d'un analyseur.
